(19) Europäisches Patentamt
European Patent Office
Office européen des brevets

(11) Numéro de publication: **0 486 595 B1**

# (12) FASCICULE DE BREVET EUROPEEN

(45) Date de publication de fascicule du brevet: **08.02.95**

(51) Int. Cl.$^6$: **A61K 7/48**, A61K 35/78

(21) Numéro de dépôt: **90912872.0**

(22) Date de dépôt: **14.08.90**

(86) Numéro de dépôt internationale :
**PCT/FR90/00610**

(87) Numéro de publication internationale :
**WO 91/02516 (07.03.91 91/06)**

(54) **Utilisation d'un extrait de coleus pour la pigmentation de la peau.**

(30) Priorité: **17.08.89 FR 8910985**

(43) Date de publication de la demande:
**27.05.92 Bulletin 92/22**

(45) Mention de la délivrance du brevet:
**08.02.95 Bulletin 95/06**

(84) Etats contractants désignés:
**AT BE CH DE DK ES FR GB IT LI LU NL SE**

(56) Documents cités:
**EP-A- 0 293 837**
**GB-A- 1 550 410**
**GB-A- 1 589 326**

(73) Titulaire: **LVMH RECHERCHE**
**48-50, rue de Seine,**
**B.P. 79**
**F-92703 Colombes Cédex (FR)**

(72) Inventeur: **MEYBECK, Alain**
**Les Poissons**
**20 ter, rue de Bezons**
**F-92400 Courbevoie (FR)**
Inventeur: **BONTE, Frédéric**
**5, place Charras**
**F-92400 Courbevoie (FR)**
Inventeur: **DUMAS, Marc**
**54, rue de l'Industrie**
**F-92700 Colombes (FR)**
Inventeur: **ANDRE, Patrice**
**9, rue Charles**
**Vappereau**
**F-45170 Neuvilles-aux-Bois (FR)**
Inventeur: **REDZINIAK, Gérard**
**101, rue des Fauvettes**
**F-45590 Saint-Cyr-en-Val (FR)**

(74) Mandataire: **Portal, Gérard et al**
**Cabinet Beau de Loménie**
**158, rue de l'Université**
**F-75340 Paris Cédex 07 (FR)**

EP 0 486 595 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne essentiellement une composition cosmétique ou pharmaceutique, notamment dermatologique, contenant un extrait de Coleus pour la pigmentation de la peau, ou en combinaison avec divers principes actifs pour la pigmentation de la peau et des cheveux, et procédé pour sa préparation.

Elle concerne également l'utilisation d'un extrait de Coleus pour la préparation d'une telle composition cosmétique ou pharmaceutique pour la pigmentation de la peau et/ou des cheveux.

Les différentes espèces de Coleus font partie de la famille des Labiacées, tout comme les Plectranthus qui en sont très proches, et sont souvent confondues entre elles, étant de plus généralement originaires des mêmes régions. Ainsi, l'espèce Coleus barbatus est généralement désignée sous le nom de Plectranthus barbatus, et l'espèce Coleus aromaticus a pour autre nom Plectranthus aromaticus. Toutefois L.H. CRAMER, dans son article publié dans Kew Bull. 1978 volume 32 n° 3 pages 551-561, discute des différences entre ces deux groupes d'espèces.

Il existe près de 200 espèces de Coleus réparties dans les régions tropicales et subtropicales d'Asie, d'Afrique, d'Australie et des îles pacifiques. Environ 9 espèces sont répertoriées en Inde. Les 10 variétés principales de Coleus sont répertoriées dans divers dictionnaires indiens, notamment : "The Wealth of India, a dictionary of Indian Raw Materials and Industrial Products, Raw Materials", volume II, Dehli 1950, pages 308-309; le livre intitulé "Indian Materia Medica" du Docteur K.M. Nadkarni's, troisième édition révisée et complétée par A.K. Nadkarni en deux volumes, volume I, page 372 ; le livre intitulé "the Flora of British India" par Sir J.D. Hooker, C.B., K.C., S.I. Volume IV ayant pour titre "Asclepiadae to Amarantaceae", pages 624 à 627.

Plus récemment, il a été mis en évidence dans les extraits de Coleus, en particulier dans les extraits de Coleus forskohlii (syn. Coleus barbatus), des substances présentant le squelette carboné des labdanes, et plus précisément, celui de la 8,13-époxylabd-14-en-11-one ayant la formule suivante :

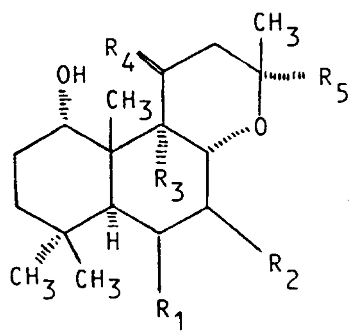

(I)

En particulier, on a isolé le dérivé 1$\alpha$,6$\beta$,9$\alpha$-trihydro-7$\beta$-acétoxy, ou forskoline, dont le procédé d'extraction a été décrit dans la demande de brevet Hoechst FR-2 336 138. On a également décrit et isolé le dérivé 1$\alpha$,7$\beta$,9$\alpha$-trihydroxy-6$\beta$-acétoxy, ou coleforsine (HOECHST, FR-2 364 211) et le dérivé 1$\alpha$,6$\beta$-dihydroxy-7$\beta$-acétoxy, ou 9-désoxyforskoline (HOECHST, EP-A1-0 243 646).

Ces documents décrivent un certain nombre de propriétés pharmacologiques de ces substances, telles qu'une activité hypotensive et calmante du système nerveux central.

On connaît également par le document EP-A-0 293 837 une composition de traitement des cheveux comprenant une combinaison d'au moins un composé choisi parmi le groupe consistant de la forskoline et de ses dérivés et de peptides ayant une structure du squelette basique, seul ou combiné avec un composé choisi parmi des acides aliphatiques, des alcools et des dérivés ayant un nombre impair d'atomes de carbone. Cette composition est appliquée sur les cheveux dans le but d'activer les mélanocytes du radix pileux et d'améliorer la synthèse de mélanine de ceux-ci en prévenant ainsi l'apparition des cheveux gris et en favorisant la restauration de la couleur naturelle.

Il est à noter, qu'entre les mélanocytes présents dans les follicules pileux et ceux présents dans la peau, il existe des différences importantes, notamment sur le plan métabolique.

De ce fait, il n'y a pas forcément de relation du point de vue de l'activité entre un composé activant les mélanocytes des follicules pileux et un composé activant les mélanocytes présents dans la peau.

La présente invention est basée sur la découverte que les extraits de Coleus, notamment de Coleus forskohlii présentent des propriétés biologiques intéressantes utilisables dans les domaines cosmétique et pharmaceutique en présentant, de façon inattendue, une activité stimulante de la mélanogénèse au niveau

des mélanocytes présents dans la peau, en permettant ainsi de favoriser la pigmentation de la peau, ainsi que de réaliser un traitement des désordres de pigmentation de la peau en favorisant plus particulièrement la biosynthèse de mélanine.

La présente invention a donc pour but de résoudre le nouveau problème technique consistant en la fourniture d'une nouvelle composition cosmétique ou pharmaceutique, notamment dermatologique, destinée à favoriser la pigmentation de la peau.

La présente invention a encore pour but de résoudre le nouveau problème technique consistant en la préparation d'une nouvelle formulation d'une composition cosmétique ou pharmaceutique présentant une bonne activité de stimulation de la mélanogénèse au niveau des mélanocytes présents dans la peau.

La présente invention a encore pour but de fournir une solution au nouveau problème technique consistant en la fourniture d'un extrait de plante particulièrement aisé à obtenir, qui présente en lui-même une bonne activité stimulante de la mélanogénèse au niveau des mélanocytes présents dans la peau, sans avoir à réaliser l'isolation d'une substance active quelconque, ces procédés d'isolation étant en général longs et coûteux.

La présente invention a encore pour but de résoudre le nouveau problème technique consistant en la fourniture d'une nouvelle formulation d'une composition cosmétique ou pharmaceutique présentant une bonne activité de stimulation de la mélanogénèse au niveau des mélanocytes présents dans les follicules pileux, en favorisant ainsi la pigmentation des cheveux.

Tous ces nouveaux problèmes techniques sont résolus pour la première fois par la présente invention d'une manière satisfaisante, utilisable à l'échelle industrielle.

Ainsi, selon un premier aspect, la présente invention concerne l'utilisation d'un extrait de Coleus pour la préparation d'une composition cosmétique ou pharmaceutique, notamment dermatologique, destinée à favoriser la pigmentation de la peau et/ou au traitement des désordres de pigmentation de la peau.

Selon une caractéristique particulière de l'invention, l'extrait de Coleus est un extrait de l'espèce Coleus forskohlii. De préférence il s'agit d'un extrait de racines.

Selon une autre caractéristique, on utilise un extrait organique de Coleus, de préférence de racines de Coleus, obtenu avantageusement par un procédé comprenant au moins une étape d'extraction avec un solvant choisi parmi le groupe constitué par l'acétate d'éthyle, le méthanol, l'éthanol et le dichlorométhane. D'une façon générale on peut utiliser comme solvant, des solvants organiques, tels que les hydrocarbures aromatiques, des hydrocarbures aliphatiques ou aromatiques halogénés, des éthers dialcoyliques, des dialcoyl-cétones, des alcanols, des acides carboxyliques et leurs esters ou d'autres solvants, comme le diméthylformamide, le dioxanne, le tétrahydrofuranne et le diméthylsulfoxyde. Parmi les solvants précités, des solvants préférés sont le benzène, le toluène ou le xylène, le chlorure de méthylène, le chloroforme, l'acétate d'éthyle, le méthanol ou l'éthanol. Le rapport de la matière végétale à l'agent d'extraction n'est pas critique et généralement sera compris entre 1 : 5 et 1 : 20 parties en poids, et il est préférentiellement de 1 : 10 environ. L'extraction s'effectue à des températures comprises entre la température ambiante et le point d'ébullition du solvant utilisé pour l'extraction. Une technique avantageuse d'extraction est la technique dite d'extraction au Soxhlet. Il peut être avantageux et dans certains cas nécessaire d'évaporer le solvant par exemple par lyophilisation et de reprendre les extraits bruts en vue d'une purification. Dans le cadre de la présente invention, l'extraction alcoolique est particulièrement intéressante, notamment en fin de procédure d'obtention de l'extrait en raison du caractère habituellement peu toxique des alcools. Ainsi un alcool particulièrement avantageux est l'éthanol.

Un autre solvant particulièrement avantageux est l'acétate d'éthyle, parce qu'il fournit un extrait riche en dérivés labd-14 ènes.

Des variantes particulières de procédé sont décrites dans la littérature, notamment dans les documents énoncés dans la partie introductive de la description précédente.

D'une façon générale, la concentration des extraits utilisés conformément à la présente invention, pour la préparation d'une composition cosmétique ou pharmaceutique, exprimée en poids sec est comprise entre 0,001% et 2% en poids, de préférence entre 0,01% et 0,5% en poids, par rapport au poids total de la composition.

Les compositions cosmétiques ou pharmaceutiques, notamment dermatologiques, selon la présente invention, peuvent être appliquées par voie topique pour favoriser la pigmentation de la peau, en particulier dans des compositions se présentant sous forme de crèmes, de gels ou de lotions destinées à l'application sur la peau.

Ainsi, les compositions cosmétiques ou pharmaceutiques, notamment dermatologiques, selon l'invention trouvent diverses applications en cosmétologie ou en dermatologie, en particulier lorsque l'augmentation de la pigmentation est recherchée. Par exemple, ces compositions peuvent être utilisées comme produits solaires pour accélérer ou intensifier le bronzage, ce qui, outre l'avantage esthétique souvent

recherché, permet de renforcer les défenses naturelles contre le rayonnement ultraviolet par l'augmentation du taux de mélanine dans l'épiderme. Ces compositions peuvent encore être utilisées, par exemple sous forme de crèmes, pour donner à la peau un aspect plus hâlé. Par ailleurs, en dermatologie, les compositions selon la présente invention peuvent être utilisées comme agents thérapeutiques, seules ou en association avec d'autres médicaments, en particulier en administration topique dans le traitement des disfonctionnements de la mélanogénèse.

Selon un mode de réalisation avantageux, une composition cosmétique ou pharmaceutique selon l'invention contient en outre une xanthine, en particulier l'IBMX ou la théophylline, de préférence à une concentration pondérale comprise entre 0,01% et 2%, et encore de préférence comprise entre 0,01% et 0,5%, par rapport au poids total de la composition.

Selon un autre mode de réalisation, une composition cosmétique ou pharmaceutique selon l'invention contient en outre de la tyrosine ou l'un de ses dérivés, de préférence à une concentration pondérale comprise entre 0,001% et 10%, par rapport au poids total de la composition.

Selon un deuxième aspect, la présente invention concerne encore un procédé de fabrication d'une composition cosmétique ou pharmaceutique, notamment dermatologique, destinée à favoriser la pigmentation de la peau, caractérisé en ce qu'il comprend l'incorporation d'au moins un extrait de Coleus tel que précédemment décrit dans un excipient, véhicule ou support cosmétiquement ou pharmaceutiquement acceptable.

Egalement, selon un troisième aspect, la présente invention concerne encore un procédé de traitement de la peau pour favoriser la pigmentation, caractérisé en ce qu'il comprend l'application en une quantité efficace pour réaliser une pigmentation, d'au moins un extrait de Coleus tel que précédemment décrit, incorporé dans un excipient, véhicule ou support cosmétiquement ou pharmaceutiquement acceptable.

Encore, selon un quatrième aspect, la présente invention concerne l'utilisation d'un extrait de Coleus combiné avec une quantité efficace d'au moins une autre substance active, choisié parmi les xanthines, en particulier l'IBMX ou la théophylline, la quinine ou ses dérivés, les rubéfiants, tels que le nicotinate de méthyle, un surnageant de culture de fibroblastes de papilles, des hydrolysats de kératine, des oligo-éléments tels que zinc, sélénium, cuivre, des inhibiteurs de 5 $\alpha$-réductase, tels que cyprotérone acétate, Minoxidil, acide azélaïque et ses dérivés, 4-méthyl-4-azastéroïde, en particulier la 17-$\beta$-N,N-diéthylcarbomoyl-4-méthyl-4-aza-5-$\alpha$-androstan-3-one, ou encore un extrait de Serenoa repens, pour la préparation d'une composition cosmétique ou pharmaceutique, destinée à favoriser la pigmentation des cheveux.

Selon des variantes de réalisation particulières, les combinaisons préférées concernent une combinaison d'extrait de Coleus, en particulier un extrait de Coleus forskohlii, de préférence de racine, avec une xanthine, en particulier l'IBMX ou la théophylline.

Selon un cinquième aspect, la présente invention concerne également un procédé de fabrication d'une composition cosmétique ou pharmaceutique, destinée à favoriser la pigmentation des cheveux, caractérisé en ce qu'il comprend l'incorporation d'au moins un extrait de Coleus tel que précédemment décrit en combinaison avec au moins une autre substance active telle qu'elle vient d'être définie ci-dessus dans le cadre de l'utilisation, dans un excipient, véhicule ou support cosmétiquement ou pharmaceutiquement acceptable.

Enfin, selon un dernier aspect, la présente invention concerne encore un procédé de traitement des cheveux pour prévenir l'apparition des cheveux gris et/ou restaurer la couleur naturelle des cheveux, caractérisé en ce qu'il comprend l'application sur les cheveux d'une quantité efficace pour réaliser une pigmentation des cheveux, d'au moins un extrait de Coleus tel que précédemment défini, combiné avec au moins une substance active telle que précédemment définie, incorporée dans un excipient, véhicule ou support cosmétiquement ou pharmaceutiquement acceptable.

Pour chaque aspect précédent pour favoriser la pigmentation des cheveux la concentration d'utilisation est généralement la même que celle qui est utilisée pour pigmenter la peau.

D'autres buts, caractéristiques et avantages de l'invention apparaîtront clairement à la lumière de la description explicative qui va suivre faite en référence à plusieurs exemples donnés simplement à titre d'illustration et qui ne saurait donc en aucune façon limiter la portée de l'invention.

Dans les exemples, les pourcentages sont donnés en poids, sauf indication contraire.

EXEMPLE 1

Obtention d'un extrait de Coleus à partir de Coleus Forskohlii

On extrait deux fois avec à chaque fois 20 l d'éther de pétrole 12 kg de racines séchées et broyées de Coleus forskohlii. On reprend les racines ainsi extraites avec à chaque fois 15 l de chlorure de méthylène

jusqu'à ce que l'extraction soit complète. On utilise en tout 60 l de chlorure de méthylène.

On réunit les extraits dans le chlorure de méthylène puis on les filtre ou on les centrifuge et on les concentre sous pression réduite.

On procède ensuite à une extraction par deux fois du résidu obtenu avec à chaque fois 3 l de méthanol.

On filtre les extraits méthanoliques réunis et on évapore le filtrat méthanolique sous pression réduite en obtenant ainsi l'extrait de Coleus forskohlii appelé extrait $I_1$.

EXEMPLE 2

On extrait deux fois 12 kg de racines séchées et broyées de Coleus forskohlii avec 15 l d'éther de pétrole.

On reprend les racines pour procéder à une extraction à plusieurs reprises avec 25 l de méthanol jusqu'à extraction complète. On filtre les extraits méthanoliques réunis et on les évapore sous vide.

On obtient ainsi un extrait de Coleus dénommé extrait $I_2$.

EXEMPLE 3

On extrait 12 kg de plantes complètes de Coleus forkohlii désséchées et broyées à plusieurs reprises avec du méthanol en utilisant au total 100 l, jusqu'à ce que l'extraction soit complète. On filtre les extraits méthanoliques réunis et on les évapore sous vide.

On peut utiliser l'extrait de Coleus ainsi obtenu.

Cependant, selon une variante de réalisation, on réalise une séparation de partage du résidu (environ 650 g) en utilisant un mélange de solvants chloroforme/eau (2/1,5 v/v). On recueille après décantation la couche chloroformique. On extrait plusieurs fois la couche aqueuse en même temps que la couche intermédiaire à chaque fois avec 1,5 l de chloroforme et on recueille à chaque fois la couche chloroformique obtenue.

On réunit les extraits chloroformiques que l'on filtre et que l'on sèche sur sulfate de sodium anhydre pour éliminer l'eau résiduelle. On évapore sous vide. On obtient environ 300 g d'extrait de Coleus dénommé extrait I3.

EXEMPLE 4

Des racines séchées et finement broyées de Coleus forskohlii (1 kg) sont extraites à plusieurs reprises avec du benzène (3 x 5 l) de préférence selon la technique d'extraction au Soxhlet à 70°C pendant plusieurs heures. Les extraits de benzène obtenus sont filtrés et concentrés sous vide pour éliminer le benzène. L'extrait obtenu de Coleus est dénommé I4.

EXEMPLE 5

On extrait 60 g de racines séchées et broyées de Coleus forskohlii au Soxhlet avec 600 ml de solvant. L'extraction se produit à température d'ébullition du solvant avec un solvant toujours renouvelé.

Le rendement moyen d'extraction est le suivant exprimé en poids sec :

a) mélange éthanol/eau (80/20 v/v) : environ 9,2 à 9,5 g, (Extrait 5A)

b) acétate d'éthyle : environ 1,5 g, (Extrait 5B)

c) dichlorométhane : environ 4 g, (Extrait 5C).

EXEMPLE 6

Mesure de l'activité d'un extrait de Coleus forskohlii selon l'invention sur les mélanocytes en culture

Protocole :

Des mélanocytes murins sont cultivés sur un milieu approprié de manière classique.

Au jour J = 0, le produit à tester est introduit dans le milieu de culture.

Au jour J = 5, on prélève les cellules que l'on isole par centrifugation et on récupère le culot cellulaire que l'on dissout dans de la soude 0,5 N.

On procède à la lecture de la densité optique sur un spectrophotomètre à 475 nm, ce qui permet d'évaluer la quantité de mélanine formée par comparaison à la densité optique d'une solution de mélanine de concentration connue.

On procède également au comptage des cellules, et on calcule le taux de mélanine formée par cellule, par rapport à une culture témoin, sans addition de produit à tester.

Essai :

L'extrait Coleus 5B, selon l'exemple 5b est testé en comparaison avec de la forskoline seule, utilisée à concentration équivalente, et avec un témoin positif consistant en $10^{-8}$ mole de $\beta$-MSH. Ainsi, on introduit au jour J = 0 dans le milieu de culture de la forskoline à la concentration de 0,1 >g/ml.

Dans une autre culture, on introduit l'extrait de Coleus à une concentration déterminée de façon que le taux en forskoline soit également d'environ 0,1 >g/ml.

Le tableau I ci-après rassemble les résultats obtenus.

TABLEAU I

| | Témoin | Extrait Coleus | Forskoline | $\beta$-MSH |
|---|---|---|---|---|
| Mélanine formée en >g pour $10^6$ cellules | 27 ± 1 | 116 ± 2 | 92 ± 3 | 70 |
| Activité A en % | 0 | 207 | 151 | 100 |

L'activité A de la stimulation de la mélanogénèse par les produits selon l'invention est calculée par la formule suivante :

$$A = \frac{q_p - q_o}{q_+ - q_o} \times 100$$

dans laquelle les grandeurs q représentent les quantités de mélanine formée

$q_p$ = culture recevant le produit à tester

$q_+$ = culture recevant la $\beta$MSH

$q_o$ = culture témoin ne recevant aucun produit.

Il résulte clairement du tableau I que l'extrait de Coleus selon l'invention stimule la production de mélanine dans une proportion significativement plus importante que la forskoline seule, ce qui représente un résultat tout à fait inattendu pour l'homme de l'art.

Par ailleurs, on a testé dans des conditions analogues les extraits de Coleus 5A et 5C respectivement obtenus par extraction par un mélange éthanol-eau 80:20 et par le dichlorométhane. On notera toutefois qu'il a été pris comme témoin positif une culture recevant de la $\beta$-MSH à la concentration de $2.10^{-8}$ M.

L'activité de ces extraits, calculée selon la formule précitée, figure au tableau II ci-après.

TABLEAU II

| Produit testé | Concentration | Mélanine formée en >g pour $10^6$ cellules | Activité A en % |
|---|---|---|---|
| Extrait 5A | 1 >g/ml | 121 ± 3 | 46 |
| Extrait 5A | 2,5 >g/ml | 161 ± 5 | 83 |
| Extrait 5A | 5 >g/ml | 188 ± 9 | 108 |
| Extrait 5B | 1 >g/ml | 89 ± 3 | 23 |
| Extrait 5B | 2,5 >g/ml | 114 ± 7 | 45 |
| Extrait 5B | 5 >g/ml | 171 ± 1 | 95 |
| Extrait 5B | 10 >g/ml | 197 ± 6 | 118 |
| $\beta$-MSH | $2.10^{-8}$ M | 179 ± 4 | 100 |
| témoin (aucun produit) | | 71 ± 3 | 0 |

EXEMPLE 7

Mesure de l'activité d'une combinaison selon l'invention de Coleus forskohlii avec la 3-isobutyl-1-méthylxanthine (IBMX), sur les mélanocytes en culture.

On opère comme à l'exemple 6. La combinaison IBMX plus extrait de Coleus est testée en comparaison avec l'IBMX et l'extrait de Coleus seul, de même qu'avec la $\beta$-MSH à la concentration de $2.10^{-8}$ M prise comme témoin positif.

Les résultats au jour J = 4 et J = 5 figurent au tableau III ci-après.

On observe au jour J = 4 et J = 5 pour la combinaison IBMX + extrait de Coleus une activité beaucoup plus importante, qui est supérieure à la somme des activités correspondant à chacun des deux constituants testés séparément.

Ceci prouve un renforcement très net de l'activité de l'extrait de Coleus par l'IBMX.

TABLEAU III

| PRODUIT | concentration >g/ml | J = 4 | | J = 5 | |
|---|---|---|---|---|---|
| | | Mélanine >g pour $10^6$ mélanocytes | A | Mélanine >g pour $10^6$ mélanocytes | A |
| Aucun (témoin) | 0 | 29 +/-3 | 0 | 51 +/-1 | 0 |
| $\beta$-MSH $2.10^{-8}$ M | | 119 +/-7 | + 100 | 200 +/-2 | + 100 |
| 1/ | 10 | 64 +/-0 | + 39 | 103 +/-2 | + 35 |
| 2/ | 0,6 | 53 +/-2 | + 27 | 85 +/-4 | + 23 |
| 3/ | 10 + 0,6 | 120 +/-6 | + 101 | 186 +/-13 | + 91 |

1 : IBMX
2 : extrait de Coleus 5B
3 : IBMX + extrait de Coleus 5B
N.B. L'activité A des produits testés est calculée comme indiqué à l'exemple 6.

EXEMPLE 8

Mesure de l'activité d'un extrait de Coleus forskohlii selon l'invention sur la pigmentation cutanée chez le cobaye

Protocole :

L'étude est menée sur un lot de 10 cobayes tricolores.

Avant et pendant l'expérimentation, les flancs droit et gauche des cobayes sont soigneusement rasés, chaque jour durant les 5 premiers jours (période d'exposition aux U.V.), puis tous les 2 jours jusqu'à la fin de l'étude.

Pour chaque animal, on détermine sur chaque flanc des taches comparablement pigmentées, d'aspect le plus souvent marron clair. Sur l'un des deux flancs tirés au hasard, on applique 10 min avant l'exposition aux rayons ultraviolets 0,5 g environ de produit à tester ou de produit témoin, selon le lot, l'autre flanc étant expose "nu" à titre de contrôle.

L'application des produits à tester est effectuée dès le premier jour d'exposition, jusqu'au sacrifice de l'animal.

L'exposition aux rayonnements ultraviolets est effectuée au moyen d'un simulateur solaire délivrant 86% d'U.V.A. et 14% d'U.V.B. pendant les 5 premiers jours de l'expérimentation, à raison de 5 min le premier jour, 10 min le deuxième jour, 15 min le troisième jour et 20 min les quatrième et cinquième jours.

Les animaux sont sacrifiés 12 jours après la dernière exposition, et l'on procède à une biopsie cutanée.

On prélève ainsi un fragment de peau sur le flanc non traité mais exposé, ainsi que sur l'autre flanc traité et exposé.

On effectue ensuite un examen histologique des fragments cutanés.

Cet examen comporte : d'une part, l'étude de la mélanogénèse par la méthode Argentaffine de Fontana sur des coupes de 4 µm (Techniques d'histologie, Professeur Chevreau, Ed. Maloine, 1977, page 157), d'autre part, l'appréciation de l'épaisseur de l'épiderme sur des coupes de 4 µm colorées selon la méthode trichomique de Masson.

L'étude de l'épaisseur de l'épiderme et de l'intensité de la mélanogénèse permet d'apprécier un effet bronzant ou plus exactement l'activation du processus de mélanogénèse.

Pour étudier la mélanogénèse, on examine deux zones tirées au hasard d'une tache pigmentée, dans lesquelles on repère 25 cellules malpigiennes et on compte parmi celles-ci les mélanocytes "activés", c'est-à-dire contenant de la mélanine en amas. On exprime alors l'activation du processus de la mélanogénèse en pourcentage de cellules activées à partir de la moyenne de ces deux valeurs.

Sur ces mêmes zones, on examine la quantité de mélanine dans les autres couches de l'épiderme et on apprécie globalement cette quantité suivant une échelle à cinq valeurs variant de 0 à 4 selon que la quantité de mélanine formée est nulle, faible, moyenne, importante ou très importante.

On a regroupé au tableau IV les résultats de l'étude histologique donnant le pourcentage d'activation et permettant d'apprécier les variations de la quantité de mélanine formée (valeurs moyennes selon l'échelle définie ci-dessus), de l'épaisseur de l'épiderme (exprimée en µm).

Le produit à tester est constitué par un extrait de Coleus à l'acétate d'éthyle selon l'exemple 5b.

TABLEAU IV

| | Flanc contrôle | Flanc traité à l'extrait de Coleus |
|---|---|---|
| % Activation | 31 | 90 |
| Quantité de mélanine | 0,71 | 2,42 |
| Epaississement de l'épiderme en µm | 8,96 | 9 |

On peut constater à partir du tableau IV que l'extrait de Coleus est actif sur la mélanogénèse. La lecture des coupes histologiques des flancs traités montre un taux très important de mélanocytes activés présentant des formes rhizomiques, et une migration importante des grains de mélanines dans l'épiderme.

Ces résultats confirment donc de manière éclatante l'activité des extraits de Coleus conformes à l'invention sur la mélanogénèse dans la peau animale.

EXEMPLE 9

Mesure de l'activité d'un extrait de Coleus forskohlii selon l'invention sur la pigmentation cutanée chez l'homme

On a mesuré l'influence des produits selon l'invention sur l'apparition et l'intensité du bronzage chez l'homme.

Protocole :

L'expérimentation a été effectuée sur 12 sujets de type de peau II ou III, dont la peau ne présente pas déjà un état bronzé.

Les produits à tester ont tout d'abord été appliqués deux fois par jour (matin et soir) sur la face interne d'un avant-bras de chaque sujet, pendant une première période d'une semaine (7 jours) ; la quantité appliquée étant celle utilisée dans les conditions habituelles d'utilisation.

Puis, ces produits ont été appliqués dans les mêmes quantités, à raison d'une seule fois par jour (le matin) pendant une seconde période de deux semaines au cours de laquelle l'irradiation est réalisée.

On a déterminé, pour chaque sujet, la dose erythémale minimale (ci-après DEM) par irradiation du bras opposé à celui sur lequel les produits sont appliqués.

Les produits à tester ont été appliqués sur quatre zones distinctes de l'avant-bras. Ces quatre zones sont exposées aux U.V. fournis par un simulateur solaire une fois par jour (l'après-midi) à une dose quotidienne inférieure à la DEM. Quand un léger bronzage apparaît, on augmente la dose de radiations administrée de 5 à 10 secondes selon le type de peau, tout en évitant l'apparition d'un érythème.

Les expositions sont réalisées chaque jour jusqu'à l'obtention d'un bronzage net, soit 8 jours au total (deux fois 4 jours séparés par une période de 2 jours).

Les mesures sont réalisées sur la peau non exposée aux U.V. et sur les zones traitées.

Une note de 0 à 5 est attribuée selon l'intensité de bronzage. La plage la plus bronzée est notée 5, la plage sans bronzage est notée 0, tandis que la note attribuée pour les autres plages est fixée entre 0 et 5 selon l'intensité de bronzage. Plusieurs lectures sont effectuées par des personnes différentes et on retient la moyenne des notes attribuées. L'écart type est calculé et on effectue un test de t pour vérifier si les résultats sont significatifs.

Trois produits ont été testés :

- produit A : extrait de Coleus 5B, selon l'invention, à 0,025 % dans un excipient gélifié,
- produit B : extrait de Coleus 5B, selon t'invention, à 0,05 % dans le même excipient,
- produit C : l'excipient gélifié.

Les résultats portés au tableau V représentent pour les trois produits A, B et C la moyenne des notations de l'intensité de bronzage durant les 8 jours d'exposition aux U.V.

TABLEAU V

| Produit | Moyenne de lecture | Significativité par rapport aux U.V. seuls |
|---|---|---|
| A | 3,55 ± 0,88 | S ($\alpha$ = 0,05) |
| B | 2,85 ± 0,76 | S ($\alpha$ = 0,05) |
| C | 2,53 ± 0,58 | NS ($\alpha$ = 0,05) |
| U.V. seuls | 2,14 ± 0,67 | S (par rapport au témoin sans U.V.) |
| témoin sans U.V. | 0 ± 0 | |

Ces résultats démontrent clairement qu'un extrait de Coleus conforme à l'invention permet d'intensifier le bronzage cutané chez l'homme. Ceci confirme les résultats des tests obtenus sur mélanocyte en culture ou in vivo sur le cobaye donnés précédemment.

On donnera ci-après divers exemples de formulation de composition cosmétique ou pharmaceutique, notamment dermatologique ayant une activité dans le traitement des désordres de la pigmentation cutanée.

EXEMPLE 10

Gel bronzant pour le visage

| | |
|---|---|
| Extrait de Coleus (poids sec) selon l'exemple 1 | 0,0284 g |
| Ethanol | 40,- g |
| Eau distillée | 20,- g |
| Gel Carbopol® 940 à 1% | qsp 100 g |

EXEMPLE 11

Crème solaire bronzante

| | |
|---|---|
| Extrait de Coleus (poids sec) selon l'exemple 2 | 0,03 g |
| Stéarate d'isocétyle | 8,- g |
| Huile d'arachide hydrogénée | 10,- g |
| Huile de lanoline | 3,5 g |
| Alcool cétylique | 5,- g |
| Alcool stéarylique | 2,5 g |
| Huile de vaseline légère | 10,- g |
| Monoester phosphorique de l'alcool cétylique OE neutralisé | 3,- g |
| Octylméthoxy cinnamate | 5,- g |

Cette phase est émulsionnée avec une phase aqueuse qsp 100 g contenant :

| | |
|---|---|
| Pantothénol | 0,1 g |
| Conservateurs | 0,2 g |

EXEMPLE 12

Lotion pour renforcer la protection solaire naturelle

| | |
|---|---|
| Alcool | 42,5 g |
| Propylèneglycol | 3,- g |
| Menthol | 0,05 g |
| Hydroxypropylméthylcellulose | 1,5 g |
| Extrait de Coleus (poids sec) selon l'exemple 5b | 0,03 g |
| Excipients aqueux parfumés | qsp 100 g |

Cette lotion est appliquée localement, de préférence deux fois par jour, quotidiennement pendant 3 à 8 jours précédant les expositions prolongées au soleil.

Les applications quotidiennes peuvent être poursuivies lors de la période d'exposition.

EXEMPLE 13

Lotion tonique capillaire contre les cheveux gris

| | |
|---|---|
| Extrait de Coleus selon l'exemple 5c | 0,02 g |
| IBMX | 0,1 g |
| Alcool | 30,- g |
| Eau | 69,- g |
| Excipients parfumés | qsp 100 g |

Cette lotion peut être appliquée sur les cheveux et le cuir chevelu deux fois par jour par cures de trois mois.

EXEMPLE 14

Gel dermatologique destiné à favoriser la pigmentation de la peau

| | |
|---|---|
| Extrait de Coleus selon l'exemple 5b | 0,03 g |
| Ethanol | 30 g |
| Eau distillée | 20 g |
| Gel de Carbopol® | qsp 100 g |

Ce gel est utilisé 1 à 2 fois par jour en application locale sur les zones de la peau à traiter.

EXEMPLE 15

Lotion capillaire contre les cheveux gris

| | |
|---|---|
| Extrait de Coleus, selon l'exemple 5b | 0,03 g |
| L-tyrosine éthyl ester, HCl | 1,0 g |
| Ethanol | 40,0 g |
| Parfum | 0,5 g |
| Cremophor | 0,2 g |
| Eau qsp | 100 g |

Cette lotion, appliquée sur les cheveux et le cuir chevelu quotidiennement, permet de retarder l'apparition de cheveux gris.

EXEMPLE 16

Gel bronzant pour la peau

| | |
|---|---|
| Extrait de Coleus | 0,03 % |
| IBMX | 0,1 % |
| Ethanol absolu | 30 % |
| Eau | 19,86 % |
| Gel de Carbopol 940® à 1,25% qsp | 100 % |

Application : 1 fois par jour sur les zones de la peau à traiter.

EXEMPLE 17

Gel traitant préventif de l'apparition des cheveux gris

| | | |
|---|---|---|
| Extrait Coleus | 0,025 % | ) A |
| Ethanol absolu | 30% | ) |
| Eau bidistillée | 19,925 % | ) B |
| Nicotinate de Méthyle | 0,05% | ) |
| Gel de Carbopol 940® à 1,25% qsp | 100 % | |

Après solubilisation des principes actifs dans A et B. Introduire A dans B, agiter 30 min à TA au barreau magnétique. Ajouter le gel, homogénéiser au Raynerie.
A appliquer quotidiennement sur les cheveux et le cuir chevelu.

EXEMPLE 18

Gel favorisant la pigmentation cutanée

| | | |
|---|---|---|
| Extrait de Coleus | 0,01 % | ) A |
| Ethanol absolu | 28 % | ) |
| Théophylline | 0,01% | ) B |
| Eau qsp | 100 % | ) |
| Carbopol 940® | 1 % | |

Ce gel est préparé selon la procédure de l'exemple 17 et appliqué 1 ou 2 fois par jour sur les zones de la peau à traiter.

Naturellement, l'invention comprend tous les moyens constituant des équivalents techniques des moyens décrits ainsi que leurs diverses combinaisons.

**Revendications**

**Revendications pour les Etats contractants suivants : AT, BE, CH, DE, DK, FR, GB, IT, LI, LU, NL, SE**

1. Utilisation d'un extrait de Coleus pour la préparation d'une composition cosmétique ou pharmaceutique destinée à favoriser la pigmentation de la peau et/ou au traitement des désordres de pigmentation de la peau.

2. Utilisation selon la revendication 1, caractérisée en ce que l'extrait de Coleus précité est un extrait de l'espère Coleus forskohlii, de préférence un extrait de racines.

3. Utilisation selon la revendication 1 ou 2, caractérisée en ce que l'extrait de Coleus précité est un extrait organique de Coleus, de préférence obtenu par un procédé comprenant au moins une étape d'extraction avec un solvant choisi parmi le groupe constitué par l'acétate d'éthyle, le méthanol, l'éthanol ou le dichlorométhane.

4. Utilisation selon l'une quelconque des revendications 1 à 3, caractérisée en ce que la concentration en extrait de Coleus précité, exprimée en poids sec, est comprise entre 0,001% et 2% en poids, de préférence entre 0,01% et 0,5% en poids, par rapport au poids total de la composition.

5. Utilisation selon l'une des revendications précédentes, caractérisée en ce qu'elle contient en outre une xanthine, en particulier l'IBMX ou la théophylline, de préférence à une concentration pondérale comprise entre 0,01 et 2%, et encore de préférence comprise entre 0,1% et 0,5%, par rapport au poids total de la composition.

6. Utilisation selon l'une des revendications précédentes, caractérisée en ce qu'elle contient en outre de la tyrosine ou l'un de ses dérivés, de préférence à une concentration pondérale comprise entre 0,001 et 10%, par rapport au poids total de la composition.

7. Utilisation selon l'une des revendications précédentes, caractérisée en ce qu'elle est formulée sous une forme permettant une application topique, en particulier sous forme de crème, de gel, destinée à l'application sur la peau.

8. Utilisation d'un extrait de Coleus combiné avec une quantité efficace d'au moins une autre substance active, choisie parmi les xanthines, en particulier l'IBMX ou la théophylline, la quinine ou ses dérivés, les rubéfiants, tels que le nicotinate de méthyle, un surnageant de culture de fibroblastes de papilles, des hydrolysats de kératine, des oligo-éléments tels que zinc, sélénium, cuivre, des inhibiteurs de 5 $\alpha$-réductase, tels que cyprotérone acétate, Minoxidil, acide azélaïque et ses dérivés, 4-méthyl-4-azastéroïde, en particulier la 17-$\beta$-N,N-diéthylcarbomoyl-4-méthyl-4-aza-5-$\alpha$-androstan-3-one, ou encore un extrait de Serenoa repens, pour la préparation d'une composition cosmétique ou pharmaceutique, destinée à favoriser la pigmentation des cheveux.

9. Utilisation selon la revendication 8, caractérisée en ce que l'extrait de Coleus précité est un extrait de l'espèce Coleus forskohlii, de préférence de racines.

10. Utilisation selon la revendication 8 ou 9, caractérisée en ce que la concentration en extrait de Coleus précité, exprimée en poids sec, est comprise entre 0,001% et 2% en poids, de préférence entre 0,01% et 0,5% en poids, par rapport au poids total de la composition.

**Revendications pour l'Etat contractant suivant : ES**

1. Utilisation d'un extrait de Coleus pour la préparation d'une composition cosmétique ou pharmaceutique destinée à favoriser la pigmentation de la peau et/ou au traitement des désordres de pigmentation de la peau.

2. Utilisation selon la revendication 1, caractérisée en ce que l'extrait de Coleus précité est un extrait de l'espère Coleus forskohlii, de préférence un extrait de racines.

3. Utilisation selon la revendication 1 ou 2, caractérisée en ce que l'extrait de Coleus précité est un extrait organique de Coleus, de préférence obtenu par un procédé comprenant au moins une étape d'extraction avec un solvant choisi parmi le groupe constitué par l'acétate d'éthyle, le méthanol, l'éthanol ou le dichlorométhane.

4. Utilisation selon l'une quelconque des revendications 1 à 3, caractérisée en ce que la concentration en extrait de Coleus précité, exprimée en poids sec, est comprise entre 0,001% et 2% en poids, de préférence entre 0,01% et 0,5% en poids, par rapport au poids total de la composition.

5. Utilisation selon l'une des revendications précédentes, caractérisée en ce qu'elle contient en outre une xanthine, en particulier l'IBMX ou la théophylline, de préférence à une concentration pondérale comprise entre 0,01 et 2%, et encore de préférence comprise entre 0,1% et 0,5%, par rapport au poids total de la composition.

6. Utilisation selon l'une des revendications précédentes, caractérisée en ce qu'elle contient en outre de la tyrosine ou l'un de ses dérivés, de préférence à une concentration pondérale comprise entre 0,001 et

10%, par rapport au poids total de la composition.

7. Utilisation selon l'une des revendications précédentes, caractérisée en ce qu'elle est formulée sous une forme permettant une application topique, en particulier sous forme de crème, de gel, destinée à l'application sur la peau.

8. Procédé de fabrication d'une composition cosmétique ou pharmaceutique, notamment dermatologique, destinée à favoriser la pigmentation de la peau, caractérisé en ce qu'il comprend l'incorporation d'au moins un extrait de Coleus dans un excipient, véhicule ou support cosmétiquement ou pharmaceutiquement acceptable.

9. Procédé selon la revendication 8, caractérisé en ce que l'extrait de Coleus précité est un extrait de l'espèce Coleus forskohlii, de préférence un extrait de racine.

10. Procédé selon la revendication 8 ou 9, caractérisé en ce que l'extrait de Coleus précité est un extrait organique de Coleus, de préférence obtenu par un procédé comprenant au moins une étape d'extraction avec l'acétate d'éthyle.

11. Procédé selon l'une des revendications 8 à 10, caractérisé en ce que l'on incorpore en outre une quantité efficace d'au moins un composé choisi parmi les xanthines, en particulier l'IBMX ou la théophylline, la tyrosine ou l'un de ses dérivés.

12. Procédé selon l'une des revendications 8 à 10, caractérisé en ce que la concentration en extrait de Coleus précité, exprimée en poids sec, est comprise entre 0,001% et 2% en poids, de préférence entre 0,01% et 0,5% en poids, par rapport au poids total de la composition.

13. Utilisation d'un extrait de Coleus combiné avec une quantité efficace d'au moins une autre substance active, choisie parmi les xanthines, en particulier l'IBMX ou la théophylline, la quinine ou ses dérivés, les rubéfiants, tels que le nicotinate de méthyle, un surnageant de culture de fibroblastes de papilles, des hydrolysats de kératine, des oligo-éléments tels que zinc, sélénium, cuivre, des inhibiteurs de 5 $\alpha$-réductase, tels que cyprotérone acétate, Minoxidil, acide azélaïque et ses dérivés, 4-méthyl-4-azastéroïde, en particulier la 17-$\beta$-N,N-diéthylcarbomoyl-4-méthyl-4-aza-5-$\alpha$-androstan-3-one, ou encore un extrait de Serenoa repens, pour la préparation d'une composition cosmétique ou pharmaceutique, destinée à favoriser la pigmentation des cheveux.

14. Utilisation selon la revendication 13, caractérisée en ce que l'extrait de Coleus précité est un extrait de l'espèce Coleus forskohlii, de préférence de racines.

15. Procédé de fabrication d'une composition cosmétique ou pharmaceutique, destiné à favoriser la pigmentation des cheveux, caractérisé en ce qu'il comprend l'incorporation d'au moins un extrait de Coleus, en particulier un extrait de l'espèce Coleus forskohlii, de préférence de racines, combiné à une quantité efficace d'au moins une autre substance active, choisie parmi les xanthines, en particulier l'IBMX ou la théophylline, la quinine ou ses dérivés, les rubéfiants, tels que le nicotinate de méthyle, un surnageant de culture de fibroblastes de papilles, des hydrolysats de kératine, des oligo-éléments tels que zinc, sélénium, cuivre, des inhibiteurs de 5 $\alpha$-réductase, tels que cyprotérone acétate, Minoxidil, acide azélaïque et ses dérivés, 4-méthyl-4-azastéroïde, en particulier la 17-$\beta$-N,N-diéthylcarbomoyl-4-méthyl-4-aza-5-$\alpha$-androstan-3-one, ou encore un extrait de Serenoa repens, dans un excipient, véhicule ou support cosmétiquement ou pharmaceutiquement acceptable pour une application sur les cheveux.

## Claims

### Claims for the following Contracting States : AT, BE, CH, DE, DK, FR, GB, IT, LI, LU, NL, SE

1. Use of an extract of Coleus for the preparation of a cosmetic or pharmaceutical composition intended for promoting skin pigmentation and/or the treatment of skin pigmentation disorders.

2. Use according to Claim 1, characterized in that the abovementioned extract of Coleus is an extract of the species Coleus forskohlii, preferably a root extract.

3. Use according to Claim 1 or 2, characterized in that the abovementioned extract of Coleus is an organic extract of Coleus, preferably obtained by a process comprising at least one step of extraction with a solvent chosen from the group consisting of ethyl acetate, methanol, ethanol and dichloromethane.

4. Use according to any one of Claims 1 to 3, characterized in that the concentration of the abovementioned extract of Coleus, expressed as dry weight, is between 0.001% and 2% by weight, and preferably between 0.01% and 0.5% by weight, relative to the total weight of the composition.

5. Use according to one of the preceding claims, characterized in that it contains, in addition, a xanthine, especially IBMX or theophylline, preferably at a weight concentration of between 0.01 and 2%, and still more preferably between 0.1% and 0.5%, relative to the total weight of the composition.

6. Use according to one of the preceding claims, characterized in that it contains, in addition, tyrosine or one of its derivatives, preferably at a weight concentration of between 0.001 and 10% relative to the total weight of the composition.

7. Use according to one of the preceding claims, characterized in that it is formulated in a form permitting topical application, especially in the form of a cream or gel, intended for application to the skin.

8. Use of an extract of Coleus combined with an effective amount of at least one other active substance chosen from xanthines, especially IBMX or theophylline, quinine or its derivatives, rubefacients such as methyl nicotinate, a culture supernatant of papilla fibroblasts, keratin hydrolysates, trace elements such as zinc, selenium, copper, 5α-reductase inhibitors such as cyproterone acetate, minoxidil, azelaic acid and its derivatives, 4-methyl-4-azasteroid, especially 17β-(N,N-diethylcarbamoyl)-4-methyl-4-aza-5α-androstan-3-one, or alternatively an extract of Serenoa repens, for the preparation of a cosmetic or pharmaceutical composition intended for promoting hair pigmentation.

9. Use according to Claim 8, characterized in that the abovementioned extract of Coleus is an extract of the species Coleus forskohlii, preferably a root extract.

10. Use according to Claim 8 or 9, characterized in that the concentration of the abovementioned extract of Coleus, expressed as dry weight, is between 0.001% and 2% by weight, and preferably between 0.01% and 0.5% by weight, relative to the total weight of the composition.

**Claims for the following Contracting State : ES**

1. Use of an extract of Coleus for the preparation of a cosmetic or pharmaceutical composition intended for promoting skin pigmentation and/or the treatment of skin pigmentation disorders.

2. Use according to Claim 1, characterized in that the abovementioned extract of Coleus is an extract of the species Coleus forskohlii, preferably a root extract.

3. Use according to Claim 1 or 2, characterized in that the abovementioned extract of Coleus is an organic extract of Coleus, preferably obtained by a process comprising at least one step of extraction with a solvent chosen from the group consisting of ethyl acetate, methanol, ethanol and dichloromethane.

4. Use according to any one of Claims 1 to 3, characterized in that the concentration of the abovementioned extract of Coleus, expressed as dry weight, is between 0.001% and 2% by weight, and preferably between 0.01% and 0.5% by weight, relative to the total weight of the composition.

5. Use according to one of the preceding claims, characterized in that it contains, in addition, a xanthine, especially IBMX or theophylline, preferably at a weight concentration of between 0.01 and 2%, and still more preferably between 0.1% and 0.5%, relative to the total weight of the composition.

6. Use according to one of the preceding claims, characterized in that it contains, in addition, tyrosine or one of its derivatives, preferably at a weight concentration of between 0.001 and 10% relative to the total weight of the composition.

7. Use according to one of the preceding claims, characterized in that it is formulated in a form permitting topical application, especially in the form of a cream or gel, intended for application to the skin.

8. Process for the manufacture of a cosmetic or pharmaceutical, in particular dermatological, composition intended for promoting skin pigmentation, characterized in that it comprises the incorporation of at least one extract of Coleus in a cosmetically or pharmaceutically acceptable excipient, vehicle or carrier.

9. Process according to Claim 8, characterized in that the abovementioned extract of Coleus is an extract of the species Coleus forskohlii, preferably a root extract.

10. Process according to Claim 8 or 9, characterized in that the abovementioned extract of Coleus is an organic extract of Coleus, preferably obtained by a process comprising at least one step of extraction with ethyl acetate.

11. Process according to one of Claims 8 to 10, characterized in that an effective amount of at least one compound chosen from xanthines, especially IBMX or theophylline, tyrosine or one of its derivatives, is incorporated in addition.

12. Process according to one of Claims 8 to 10, characterized in that the concentration of the abovementioned extract of Coleus expressed as dry weight, is between 0.001% and 2% by weight, and preferably between 0.01% and 0.5% by weight, relative to the total weight of the composition.

13. Use of an extract of Coleus combined with an effective amount of at least one other active substance chosen from xanthines, especially IBMX or theophylline, quinine or its derivatives, rubefacients such as methyl nicotinate, a culture supernatant of papilla fibroblasts, keratin hydrolysates, trace elements such as zinc, selenium, copper, 5α-reductase inhibitors such as cyproterone acetate, minoxidil, azelaic acid and its derivatives, 4-methyl-4-azasteroid, especially 17β-(N,N-diethylcarbamoyl)-4-methyl-4-aza-5α-androstan-3-one, or alternatively an extract of Serenoa repens, for the preparation of a cosmetic or pharmaceutical composition intended for promoting hair pigmentation.

14. Use according to Claim 13, characterized in that the abovementioned extract of Coleus is an extract of the species Coleus forskohlii, preferably a root extract.

15. Process for the manufacture of a cosmetic or pharmaceutical composition intended for promoting hair pigmentation, characterized in that it comprises the incorporation of at least one extract of Coleus, especially an extract of the species Coleus forskohlii, preferably a root extract, combined with an effective amount of at least one other active substance chosen from xanthines, especially IBMX or theophylline, quinine or its derivatives, rubefacients such as methyl nicotinate, a culture supernatant of papilla fibroblasts, keratin hydrolysates, trace elements such as zinc, selenium, copper, 5α-reductase inhibitors such as cyproterone acetate, minoxidil, azelaic acid and its derivatives, 4-methyl-4-azasteroid, especially 17β-(N,N-diethylcarbamoyl)-4-methyl-4-aza-5α-androstan-3-one, or alternatively an extract of Serenoa repens, in a cosmetically or pharmaceutically acceptable excipient, vehicle or carrier for application to hair.

## Patentansprüche

## Patentansprüche für folgende Vertragsstaaten : AT, BE, CH, DE, DK, FR, GB, IT, LI, LU, NL, SE

1. Verwendung eines Coleus-Extrakts zur Herstellung kosmetischer oder pharmazeutischer Zusammensetzungen, die zur Förderung der Pigmentierung der Haut und/oder zur Behandlung von Pigmentanomalien der Haut bestimmt sind.

2. Verwendung nach Anspruch 1, dadurch gekennzeichnet, daß der Coleus-Extrakt ein Extrakt aus Pflanzen der Art Coleus forskolii, vorzugsweise ein Wurzelextrakt, ist.

3. Verwendung nach Anspruch 1 oder 2, dadurch gekennzeichnet, daß der Coleus-Extrakt ein organischer Coleus-Extrakt ist, der vorzugsweise durch ein Verfahren erhalten wird, das mindestens einen Extraktionsschritt mit einem Lösungsmittel umfaßt, das unter Ethylacetat, Methanol, Ethanol und Dichlormethan ausgewählt ist.

4. Verwendung nach einem der Ansprüche 1 bis 3, dadurch gekennzeichnet, daß die auf das Trockengewicht bezogene Konzentration an Coleus-Extrakt 0,001 bis 2 Gew.-%, vorzugsweise 0,01 bis 0,5 Gew.-%, bezogen auf das Gesamtgewicht der Zusammensetzung, beträgt.

5. Verwendung nach einem der vorhergehenden Ansprüche, dadurch gekennzeichnet, daß die Zusammensetzung zusätzlich ein Xanthin, insbesondere 3-Isobutyl-1-methylxanthin (IBMX) oder Theophyllin, vorzugsweise in einer Konzentration enthält, die 0,01 bis 2 Gew.-% und vorzugsweise 0,1 bis 0,5 Gew.-%, bezogen auf das Gesamtgewicht der Zusammensetzung, beträgt.

6. Verwendung nach einem der vorhergehenden Ansprüche, dadurch gekennzeichnet, daß die Zusammensetzung außerdem Tyrosin oder eines seiner Derivate, vorzugsweise in einer Konzentration von 0,001 bis 10 Gew.-%, bezogen auf das Gesamtgewicht der Zusammensetzung, enthält.

7. Verwendung nach einem der vorhergehenden Ansprüche, dadurch gekennzeichnet, daß die Zusammensetzung in einer Form formuliert ist, die eine topische Anwendung insbesondere in Form einer Creme oder eines Gels zur Anwendung auf der Haut ermöglicht.

8. Verwendung eines Coleus-Extrakts, der mit einer wirksamen Menge von mindestens einer weiteren wirksamen Substanz kombiniert ist, die ausgewählt ist unter Xanthinen, insbesondere IBMX und Theophyllin, Chinin und seinen Derivaten, hautreizenden Mitteln, wie z.B. Methylnicotinat, dem Überstand der Kultivierung von Fibroblasten aus Papillen, Keratinhydrolysaten, Spurenelementen, wie z.B. Zink, Selen, Kupfer, Inhibitoren der 5-$\alpha$-Reductase, wie z.B. Cyproteronacetat, Minoxidil, Azelainsäure und ihren Derivaten, 4-Methyl-4-azasteroide, insbesondere 17-$\beta$-N,N-Diethylcarbamoyl-4-methyl-4-aza-5-$\alpha$-androstan-3-on und einem Extrakt von Serenoa repens, zur Herstellung kosmetischer oder pharmazeutischer Zusammensetzungen, die zur Förderung der Pigmentierung des Haars bestimmt ist.

9. Verfahren nach Anspruch 8, dadurch gekennzeichnet, daß der Coleus-Extrakt ein Extrakt aus Pflanzen der Art Coleus forskolii, vorzugsweise ein Wurzelextrakt, ist.

10. Verwendung nach Anspruch 8 oder 9, dadurch gekennzeichnet, daß die auf das Trockengewicht bezogene Konzentration an Coleus-Extrakt 0,001 bis 2 Gew.-%, vorzugsweise 0,01 bis 0,5 Gew.-%, bezogen auf das Gesamtgewicht der Zusammensetzung, beträgt.

**Patentansprüche für folgenden Vertragsstaat : ES**

1. Verwendung eines Coleus-Extrakts zur Herstellung kosmetischer oder pharmazeutischer Zusammensetzungen, die zur Förderung der Pigmentierung der Haut und/oder zur Behandlung von Pigmentanomalien der Haut bestimmt sind.

2. Verwendung nach Anspruch 1, dadurch gekennzeichnet, daß der Coleus-Extrakt ein Extrakt aus Pflanzen der Art Coleus forskolii, vorzugsweise ein Wurzelextrakt, ist.

3. Verwendung nach Anspruch 1 oder 2, dadurch gekennzeichnet, daß der Coleus-Extrakt ein organischer Coleus-Extrakt ist, der vorzugsweise durch ein Verfahren erhalten wird, das mindestens einen Extraktionsschritt mit einem Lösungsmittel umfaßt, das unter Ethylacetat, Methanol, Ethanol und Dichlormethan ausgewählt ist.

4. Verwendung nach einem der Ansprüche 1 bis 3, dadurch gekennzeichnet, daß die auf das Trockengewicht bezogene Konzentration an Coleus-Extrakt 0,001 bis 2 Gew.-%, vorzugsweise 0,01 bis 0,5 Gew.-%, bezogen auf das Gesamtgewicht der Zusammensetzung, beträgt.

5. Verwendung nach einem der vorhergehenden Ansprüche, dadurch gekennzeichnet, daß die Zusammensetzung zusätzlich ein Xanthin, insbesondere 3-Isobutyl-1-methylxanthin (IBMX) oder Theophyllin, vorzugsweise in einer Konzentration enthält, die 0,01 bis 2 Gew.-% und vorzugsweise 0,1 bis 0,5 Gew.-%, bezogen auf das Gesamtgewicht der Zusammensetzung, beträgt.

6. Verwendung nach einem der vorhergehenden Ansprüche, dadurch gekennzeichnet, daß die Zusammensetzung außerdem Tyrosin oder eines seiner Derivate, vorzugsweise in einer Konzentration von

0,001 bis 10 Gew.-%, bezogen auf das Gesamtgewicht der Zusammensetzung, enthält.

7. Verwendung nach einem der vorhergehenden Ansprüche, dadurch gekennzeichnet, daß die Zusammensetzung in einer Form formuliert ist, die eine topische Anwendung insbesondere in Form einer Creme oder eines Gels zur Anwendung auf der Haut ermöglicht.

8. Verfahren zur Herstellung kosmetischer oder pharmazeutischer, insbesondere dermatologischer Zusammensetzungen, die zur Förderung der Pigmentierung der Haut bestimmt sind, dadurch gekennzeichnet, daß mindestens ein Coleus-Extrakt mit einem kosmetisch oder pharmazeutisch akzeptablen Excipiens, Vehikel oder Träger vermengt wird.

9. Verfahren nach Anspruch 8, dadurch gekennzeichnet, daß der Coleus-Extrakt ein Extrakt aus Pflanzen der Art Coleus forskolii, vorzugsweise ein Wurzelextrakt, ist.

10. Verfahren nach Anspruch 8 oder 9, dadurch gekennzeichnet, daß der Coleus-Extrakt ein organischer Coleus-Extrakt ist, der vorzugsweise durch ein Verfahren erhalten wird, das mindestens einen Extraktionsschritt mit Ethylacetat umfaßt.

11. Verfahren nach einem der Ansprüche 8 bis 10, dadurch gekennzeichnet, daß außerdem eine wirksame Menge von mindestens einer Verbindung eingebracht wird, die unter Xanthinen, insbesondere IBMX und Theophyllin, sowie Tyrosin und seinen Derivaten ausgewählt ist.

12. Verfahren nach einem der Ansprüche 8 bis 10, dadurch gekennzeichnet, daß die auf das Trockengewicht bezogene Konzentration an Coleus-Extrakt 0,001 bis 2 Gew.-%, vorzugsweise 0,01 bis 0,5 Gew.-%, bezogen auf das Gesamtgewicht der Zusammensetzung, beträgt.

13. Verwendung eines Coleus-Extrakts, der mit einer wirksamen Menge von mindestens einer weiteren wirksamen Substanz kombiniert ist, die ausgewählt ist unter Xanthinen, insbesondere IBMX und Theophyllin, Chinin und seinen Derivaten, hautreizenden Mitteln, wie z.B. Methylnicotinat, dem Überstand der Kultivierung von Fibroblasten aus Papillen, Keratinhydrolysaten, Spurenelementen, wie z.B. Zink, Selen, Kupfer, Inhibitoren der 5-$\alpha$-Reductase, wie z.B. Cyproteronacetat, Minoxidil, Azelainsäure und ihren Derivaten, 4-Methyl-4-azasteroide, insbesondere 17-$\beta$-N,N-Diethylcarbamoyl-4-methyl-4-aza-5-$\alpha$-androstan-3-on und einem Extrakt von Serenoa repens, zur Herstellung kosmetischer oder pharmazeutischer Zusammensetzungen, die zur Förderung der Pigmentierung des Haars bestimmt ist.

14. Verwendung nach Anspruch 13, dadurch gekennzeichnet, daß der Coleus-Extrakt ein Extrakt aus Pflanzen der Art Coleus forskolii, vorzugsweise ein Wurzelextrakt, ist.

15. Verfahren zur Herstellung kosmetischer oder pharmazeutischer Zusammensetzungen, die zur Förderung der Pigmentierung des Haars bestimmt ist, dadurch gekennzeichnet, daß es das Vermengen von mindestens einem Coleus-Extrakt, insbesondere eines Extrakts aus Pflanzen der Art Coleus forskolii, vorzugsweise eines Wurzelextrakts, in Kombination mit einer wirksamen Menge von mindestens einer weiteren wirksamen Substanz, die ausgewählt ist unter Xanthinen, insbesondere IBMX und Theophyllin, Chinin und seinen Derivaten, hautreizenden Mitteln, wie z.B. Methylnicotinat, dem Überstand der Kultivierung von Fibroblasten von Papillen, Keratinhydrolysaten, Spurenelementen, wie z.B. Zink, Selen, Kupfer, Inhibitoren der 5-$\alpha$-Reductase, wie z.B. Cyproteronacetat, Minoxidil, Azelainsäure und ihren Derivaten, 4-Methyl-4-azasteroiden, insbesondere 17-$\beta$-N,N-Diethylcarbamoyl-4-methyl-4-aza-5-$\alpha$-androstan-3-on und einem Extrakt von Serenoa repens, mit einem kosmetisch oder pharmazeutisch akzeptablen Excipiens, Vehikel oder Träger zur Anwendung auf den Haaren umfaßt.